# EUROPÄISCHE PATENTSCHRIFT

(11) **EP 0 411 277 B1**
(45) Veröffentlichungstag und Bekanntmachung des Hinweises auf die Patenterteilung: **09.11.1994**
(21) Anmeldenummer: 90110663.3
(22) Anmeldetag: 06.06.1990
(51) Int. Cl.: C07D 239/66

(54) **Verfahren zur Herstellung von Na-Thiobarbiturat**
Process for preparation of Na-Thiobarbiturate
Procédé de préparation de Thiobarbiturate de sodium

(30) Priorität: 03.08.1989 DE 3925689
(43) Veröffentlichungstag der Anmeldung: 06.02.1991
(73) Patentinhaber: HÜLS AKTIENGESELLSCHAFT, 45764 Marl (DE)
(72) Erfinder: Feld, Marcel, Dr., D-5000 Köln 90 (DE)

(56) Entgegenhaltungen:
- CH-A- 411 901
- GB-A- 770 344
- "Beilstein Handbuch der Organischen Chemie" Vol.24, 1936, Springer Verlag,Berlin
- Collection of Czechoslovak Chemical Communications vol. 45, 1980, Prague Seiten732 - 739; Jaromir Kavalek et al.: "Study of Reaction of Thiourea with Ethyl 3-Oxobutanoate"

## Beschreibung

Die vorliegende Erfindung beschreibt ein technisches Verfahren zur Herstellung des Na-Salzes der Thiobarbitursäure, aus dem mittels Mineralsäuren die Thiobarbitursäure gewonnen werden kann. Thiobarbitursäure und das entsprechende Na-Salz sind u.a. als Zwischenprodukte für die Herstellung von Insektiziden oder Pestiziden von Interesse.

Das erfindungsgemäße Verfahren beruht auf der Umsetzung von Malonsäuredimethylester mit jeweils annähernd äquimolaren Mengen Thioharnstoff und Natriummethylat in Methanol als Lösungsmittel bei erhöhter Temperatur, vorzugsweise bei Rückflußtemperatur unter Normaldruck.

Aus der Literatur (J. pr. Chem. 35, 456; J. Org. Chem. 26, (1961) 792) ist die Herstellung von Na-Thiobarbiturat und der daraus erhältlichen Thiobarbitursäure durch Umsetzung von Thioharnstoff mit dem Na-Salz des Malonsäurediethylesters in ethanolischem Medium bekannt, wonach Thiobarbitursäure in unbefriedigender Ausbeute von 84 % d.Th. erhalten wurde. Auch ausgehend von Thioharnstoff, Malonsäurediethylester und Alkalialkoholat ist die herstellung von Thiobarbitursäure bekannt. In Abhängigkeit vom verwendeten Alkalialkoholat wurden dabei unterschiedliche Ausbeuten erhalten, die im Falle des Natriumethylats 60 %, im Falle des preiswerteren Natriummethylats gar nur 37 % betrugen (Sbornik Statei Obshchei Khim. 2, 1273-4 (1953)). Neben der völlig unbefriedigenden Ausbeute hätte ein derartiges Herstellungsverfahren noch den Nachteil, daß dabie Ethanol-Methanol-Gemische resultieren, die einen zusätzlichen Aufarbeitungsschritt erfordern würden.

Es bestand somit die Aufgabe, Natriumthiobarbiturat nach einem technisch einfachen Verfahren unter Verwendung des preiswerten Natriummethylats in hoher Ausbeute herzustellen.

Aus einer rein kinetischen Studie ist bereits die Umsetzung von Thioharnstoff mit Malonsäuredimethylester und Natriummethylat in methanolischer Lösung bekannt (Collection Czchoslov. Chem. Commun., Vol. 45, 734 (1980)). Da dabie die beiden Komponenten Malonsäuredimethylester und Natriummethylat gegen Thioharnstoff in einem dreihuntert- bis sechshuntertfachen molaren Überschuß eingesetzt wurden, verstärkte diese Studie noch den aus der vorgenannten Literatur vermittelten Eindruck, daß ein technisches Herstellungsverfahren für Na-Thiobarbiturat auf der Basis dieser Ausgangsprodukte wenig Aussicht auf Erfolg haben sollte.

Entgegen dieser aus der Literatur zu entnehmenden Lehre kann gemäß dem erfindungsgemäßen Verfahren Na-Thiobarbiturat in hohen Ausbeute nach einem technisch einfachen Verfahren durch Umsetzung von Thioharnstoff, Malonsäuredimethylester und Natriummethylat in methanolischer Lösung hergestellt werden.

Gegenstand der Erfindung ist das Verfahren nach dem Patentanspruch und den Unteransprüchen. Die drei Ausgangsstoffe werden in annähernd molarem Verhältnis 1 : 1 : 1 eingesetzt. Überschüsse bis 100 Mol-% besonders bis 25 Mol-% der Komponenten sind möglich, aber ohne Vorteile. Die Umsetzung wird bei erhöhten Temperaturen, vorzugsweise zwischen 40 und 120 °C, besonders bevorzugt unter Rückflußbedingungen des Methanols bei Normaldruck durchgeführt.

Nach einer bevorzugten Verfahrensvariante wird das nach beendeter Reaktion und Isolierung des Zielproduktes durch eine übliche Fest-Flüssig-Trennung anfallende methanolische Filtrat ganz oder zumindest größtenteils wiederholt als Reaktionsmedium für weitere Umsetzungen von Malonsäuredimethylester mit Thioharnstoff und Natriummethylat eingesetzt.

Die Menge des als Reaktionsmedium verwendeten Methanols kann beim erfindungsgemäßen Verfahren in weiten Grenzen variiert werden. Im Hinblick auf eine bestmögliche Nutzung der technischen Apparate ist jedoch eine relativ geringe Lösemittelmenge sinnvoll. Bezogen auf 1 Gew.-Teil Thioharnstoff werden daher vorzugsweise 2 bis 4 Gewichtsteile Methanol eingesetzt. Eine Teilmenge des Methanols kann dabei in Form einer methanolischen Lösung des Natriummethylats zum Einsatz gebracht werden.

Die Reihenfolge, in der die Ausgangsstoffe Malonsäuredimethylester, Thioharnstoff und Natriummethylat zugegeben werden, ist ohne entscheidende Bedeutung. Bei der technischen Durchführung kann es jedoch von praktischem Vorteil sein, den festen Thioharnstoff in Methanol gelöst vorzulegen und dieser Lösung nacheinander oder gleichzeitig den Malonester und eine methanolische Lösung des Natriummethylats zuzufügen. Die Zugabe einer oder zweier der drei Komponenten kann auch erst unter Reaktionsbedingungen portionsweise oder kontinuierlich erfolgen.

Bereits bei Einsatz der Ausgangsstoffe in äquimolaren Verhältnissen wird beim erfindungsgemäßen Verfahren eine hohe Ausbeute an Zielprodukt erreicht. Im Falle des Natriummethylats kann ein Überschuß jedoch dann von Nachteil sein, wenn das Na-Thiobarbiturat nicht zu Thiobarbitursäure weiterverarbeitet, sondern beispielsweise zu 2-(Methylthio)barbitursäure methyliert werden soll. Da Thiobarbitursäure gegenüber Natriummethylat als mehrbasige Säure fungiert, bildet sich mit dem Überschuß der Base auch ein Na₂-Salz der Thiobarbitursäure, das beispielsweise bei Alkylierungen zu dialkylierten Produkten führen kann.

Zur Aufarbeitung des beim erfindungsgemäßen Verfahren resultierenden Reaktionsgemisches wird das gebildete, in Methanol schwer lösliche Natriumthiobarbiturat durch eine übliche Fest/Flüssig-Trennung isoliert und gegebenenfalls mit Methanol gewaschen. Das Filtrat kann teilweise oder ganz, ggf. nach Einengung auf ein gewünschtes Volumen, wiederholt als Reaktionsmedium für weitere Umsetzungen verwendet werden. Eine mehrfache derartige Wiederverwendung der methanolischen Mutterlauge vermindert nicht nur drastisch die aufzuarbeitenden bzw. zu entsorgenden Lösungs- bzw. Rückstandsmengen aus der Reaktion, sondern führt auch zu einer deutlichen Verbesserung der Gesamtausbeute auf über 98 %, wobei überraschend sehr gute Reinheiten im Bereich von 99,8 bis 99,9 % erreichbar sind.

Bei Wiedereinsatz von methanolischer Mutterlauge und Waschfiltrat kann eine Einengung auch erst während der Reaktion durch Abdestillation von Methanol vorgenommen werden. Dadurch kann auf bequeme Weise die durch die vorausgegangene Kondensationsreaktion und das Waschmethanol einerseits und das ggf. als Lösungsmittel für Natriummethylat gegenüber dem vorausgegangenen Ansatz vermehrte Methanolmenge ausgeglichen und so für stets gleiche Konzentrationsverhältnisse beim aufzuarbeitenden Reaktionsgemisch gesorgt werden.

### Beispiel 1

Eine Lösung von 150,7 g (2,0 Mol) Thioharnstoff und 237,8 g (1,8 Mol) Malonsäuredimethylester in 400 g Methanol wurde zum Rückfluß erhitzt und innerhalb von 15 Min. eine Lösung von 97 g (1,8 Mol) NaOCH₃ in 224 g Methanol zugesetzt. Nach weiteren 4 Std. unter Rückfluß wurde auf Raumtemperatur abgekühlt, filtriert, der Filterkuchen mit 100 g Methanol gewaschen und getrocknet. Es resultierten 278 g (93 % d.Th. bezogen auf Malonester) Natriumthiobarbiturat als weißes Kristallisat mit einer Reinheit von 99,8 %.

Die methanolischen Filtrate wurden auf 400 g eingeengt, zunächst 137 g (1,8 Mol) Thioharnstoff und 237,8 g (1,8 Mol) Malonsäuredimethylester, später unter Rückflußbedingungen wiederum 97 g (1,8 Mol) Natriummethylat in 224 g Methanol zugefügt und in der zuvor beschriebenen Weise weiterverfahren. In gleicher Weise wurden die jeweils auf 400 g eingeengten methanolischen Filtrate zum Umsatz von je 137 g Malonsäuredimethylester und 97 g Natriummethylat noch weitere 2 Mal eingesetzt. Die Gesamtausbeute aller 4 Versuche Betrug 1.185 g (99,1 % d.Th.) Natriumthiobarbiturat bei einer Produktreinheit von 99,8 bis 99,9 %.

### Beispiel 2

Die in Beispiel 1 beschriebene Versuchsserie wurde wiederholt, wobei jeweils der Thioharnstoff und Natriummethylat in Methanol bzw. dem auf 400 g eingeengten methanolischen Filtrat des vorausgegangenen Versuches vorgelegt und unter Rückflußbedingungen der Malonester zugesetzt wurde. Beim Startversuch der Versuchsserie wurden 283,1 g (94,7 % d.Th.) Natriumthiobarbiturat erhalten. Als Gesamtausbeute aller 4 Versuche resultierten 1.187 g (99,2 % d.Th.) Natriumthiobarbiturat mit einer Reinheit von 99,8 bis 99,9 %.

### Beispiel 3

Die in Beispiel 1 beschriebene Versuchsserie wurde wiederholt, wobei jeweils der Malonester und Natriummethylat in Methanol bzw. dem auf 400 g eingeengten methanolischen Filtrat eines vorausgegangenen Versuches vorgelegt, innerhalb von 45 Min. bei 60 °C portionsweise mit dem Thioharnstoff versetzt und dann in der beschriebenen Weise weiterverfahren wurde. Die Ausbeute des Startversuches betrug 274,1 g (91,7 % d.Th.), die Gesamtausbeute aller 4 Versuche 1.182 g (98,9 % d.Th.) Natriumthiobarbiturat bei Reinheitsgraden von 99,7 bis 99,9 %.

## Patentansprüche

1. Verfahren zur Herstellung von Natriumthiobarbiturat durch Umsetzung von Malonsäuredimethylester, Thioharnstoff und Natriummethylat in Methanol als Reaktionsmedium bei Temperaturen von 40 bis 120°C, **dadurch gekennzeichnet,** daß auf 1 Mol einer der 3 Komponenten Thioharnstoff, Malonsäuredimethylester und Natriummethylat von den beiden anderen Komponenten 1 bis 1,25 Mol eingesetzt werden und das gebildete Natriumthiobarbiturat durch eine übliche Fest-Flüssig-Trennung isoliert wird.

2. Verfahren nach Anspruch 1, **dadurch gekennzeichnet,** daß die Umsetzung bei Rückflußtemperatur des Methanols unter Normaldruck durchgeführt wird.

3. Verfahren nach einem der Ansprüche 1 bis 2, **dadurch gekennzeichnet,** daß das nach Abtrennung des Zielproduktes erhaltene methanolische Filtrat ganz oder teilweise, ggf. nach Einengung auf ein gewünschtes Volumen mehrfach als Reaktionsmedium für weitere Umsetzungen von Thioharnstoff mit Malonsäuredimethylester und Natriummethylat verwendet wird.

4. Verfahren nach einem der Ansprüche 1 bis 3, **dadurch gekennzeichnet,** daß Methanol als Lösungsmittel in Mengen von 2 bis 4 Gewichtsteilen je Gewichtsteil Thioharnstoff eingesetzt wird.

## Claims

1. Process for the preparation of sodium thiobarbiturate by reaction of malonic acid dimethyl ester, thiourea and sodium methylate in methanol as reaction medium at temperatures of 40 to 120^{o}C, **characterised in that**, for 1 mol of one of the three components thiourea, malonic acid dimethyl ester and sodium methylate there are employed 1 to 1.25 mol of the two other components and the sodium thiobarbiturate formed is isolated by a conventional solid-liquid separation.

2. Process according to claim 1, **characterised in that** the reaction is carried out at reflux temperature of the methanol under normal pressure.

3. Process according to one of claims 1 to 2, **characterised in that** the methanolic filtrate obtained after separation off of the target product is used as reaction medium again and again completely or partially for further reactions of thiourea with malonic acid dimethyl ester and sodium methylate, possibly after concentration to a desired volume.

4. Process according to one of claims 1 to 3, **characterised in that** methanol is employed as solvent in amounts of 2 to 4 parts by weight per part by weight of thiourea.

## Revendications

1. Procédé de préparation de thiobarbiturate de sodium par la réaction de l'ester diméthylique de l'acide malonique, de la thiourée et du méthylate de sodium dans du méthanol comme milieu de réaction à des températures de 40 à 120°C, procédé caractérisé en ce qu'on utilise, pour 1 mole de l'un des trois constituants : la thiourée, l'ester diméthylique de l'acide malonique et le méthylate de sodium, 1 à 1,25 mole des deux autres constituants et en ce qu'on isole le thiobarbiturate de sodium par une séparation usuelle solide/liquide.

2. Procédé selon la revendication 1, caractérisé en ce qu'on effectue la réaction à la température de reflux du méthanol sous la pression normale.

3. Procédé selon l'une des revendications 1 ou 2, caractérisé en ce qu'après séparation du produit visé, on utilise le filtrat méthanolique obtenu, entièrement ou partiellement, éventuellement après concentration à un volume voulu, plusieurs fois comme milieu de réaction pour d'autres réactions de la thiourée avec l'ester diméthylique de l'acide malonique et le méthylate de sodium.

4. Procédé selon l'une des revendications 1 à 3, caractérisé en ce qu'on utilise le méthanol comme solvant en des quantités de 2 à 4 parties en poids pour chaque partie en poids de la thiourée.
